# EUROPEAN PATENT APPLICATION

(11) **EP 2 189 503 A1**
(43) Date of publication of application: **26.05.2010**
(21) Application number: 08711776.8
(22) Date of filing: 21.02.2008
(51) Int. Cl.: C09B 69/00, C07D 221/22, C07D 239/54, G01N 33/58

(54) **NOVEL NUCLEIC ACID DERIVATIVE INTO WHICH GIVEN COMPOUND HAS BEEN INTRODUCED AND METHOD FOR PRODUCING THE SAME**

(30) Priority: 09.08.2007 US 964105 P; 28.09.2007 JP 2007254803
(71) Applicant: Biodynamics Laboratory, INC., 9-7 Hongo 2-Chome Bunkyo-Ku Tokyo 113-0033 (JP)
(72) Inventor: KATO, Toshiyuki, Tokyo 113-0033 (JP); NAGAHORA, Hitoshi, Tokyo 113-0033 (JP); ONDA, Masaaki, Tokyo 113-0033 (JP); TANAKA, Shingo, Tokyo 113-0033 (JP)
(74) Representative: Dunleavy, Kevin James
(86) International application number: PCT/JP2008/052993
(87) International publication number: WO 2009/019901

(57) **Abstract**

The objective of the invention is to provide nucleic acid derivatives with designated compounds introduced specifically into the base of nucleic acids. A DNA or RNA, or a nucleic acid molecular weight marker, wherein a designated compound (R), which is selected from any one of dyes, fluorescent dyes, RI labeled substances, and compounds capable of linking specifically to other compounds, is introduced at the N-3 position of a thymine base or uracil base in which (R') at Position 1 is other than hydrogen atom.

SEQUENCE LISTING

<110>  BIODYNAMICS LABORATORY, INC.
       Kato, Toshiuki
       Nagahora, Hitoshi
       Onda, Masaaki
       Tanaka, Shingo

<120>  NOVEL NUCLEIC ACID DERIVATIVE INTO WHICH GIVEN COMPOUND
       HAS BEEN INTRODUCED AND METHOD FOR PRODUCING THE SAME

<130>  125192.00601

<140>  EP08711776.8
<141>  2008-02-21

<150>  US 60/964,105
<151>  2007-08-09

<150>  JP 2007-254803
<151>  2007-09-28

<160>  9

<170> PatentIn version 3.5

<210>  1
<211>  30
<212>  DNA
<213>  Artificial sequence

<220>
<223>  Synthetic DNA oligomer

<400>  1
aaaaaaaaaa aaaaaaaaaa aaaaaaaaaa     30

<210>  2
<211>  30
<212>  DNA
<213>  Artificial sequence

<220>
<223>  Synthetic DNA oligomer

<400>  2
gggggggggg gggggggggg gggggggggg     30

<210>  3
<211>  30
<212>  DNA
<213>  Artificial Sequence

<220>
<223>  Synthetic DNA oligomer

<400>  3
tttttttttt tttttttttt tttttttttt     30

<210>  4
<211>  30
<212>  DNA
<213>  Artificial sequence

<220>
<223>  synthetic DNA oligonier

<400>  4
cccccccccc cccccccccc cccccccccc    30

<210>  5
<211>  30
<212>  DNA
<213>  Artificial Sequence

<220>
<223>  Synthetic DNA oligcnier

<400>  5
uuuuuuuuuu uuuuuuuuuu uuuuuuuuuu    30

<210>  6
<211>  50
<212>  DNA
<213>  Artificial sequence

<220>
<223>  Synthetic deoxyribophosphate oligomer

<400>  6
tttttttttt tttttttttt tttttttttt tttttttttt tttttttttt    50

<210>  7
<211>  40
<212>  DNA
<213>  Artificial sequence

<220>
<223>  Synthetic deoxyribophosphate oligomer

<400>  7
tttttttttt tttttttttt tttttttttt tttttttttt    40

<210>  8
<211>  30
<212>  DNA
<213>  Artificial Sequence

<220>
<223>  Synthetic deoxyribophosphate oligomer

<400>  8
tttttttttt tttttttttt tttttttttt    30

<210>  9
<211>  20
<212>  DNA
<213>  Artificial Sequence

<220>
<223>  Synthetic deoxyribophosphate oligomer

<400>  9
tttttttttt tttttttttt    20

## Description

### CROSS REFERENCE TO RELATED APPLICATIONS

This application claims priority under EPC, Article 87 based upon US Provisional Apllication No.60/964,105, filed on August 9,2007, and Japanese Patent Application Serial No.2007-254803, filed on September 28,2007.

### FIELD OF THE INVENTION

The present invention relates to a nucleic acid derivative in which a designated compound has been introduced, and a process for the preparation thereof, and further, and a method for use thereof.

### BACKGROUND OF THE INVENTION

Nucleic acid derivatives have heretofore been extensively applied as nucleic acid drugs, such as anti-cancer agents, antiviral agents, drugs for improved brain metabolism, and the like. These nucleic acid derivatives are nucleic acids which have been modified by introduction of a designated functional compound or by substitution with other functional groups and the like, and they provide the advantages of allowing a synthesis of a nucleic acid molecule having a variety of functions depending upon the compound or functional group introduced therein and /or allowing an efficient design of a functional nucleic acid molecule specific to a target base sequence. Currently, applications making use of the advantages of these nucleic acid derivatives are being investigated in basic biological experimental techniques as nucleic acid molecular weight markers, nucleic acid probes, and the like, and in transcriptome analysis techniques such as DNA microarrays and the like.

On the other hand, there are occasions where in order to check the presence of a nucleic acid specimen or its molecular weight, it becomes necessary to visualize said nucleic acid specimen in biological experiments and the like. Such visualization methods generally involve a procedure of subjecting the nucleic acid specimen to gel electrophoresis, staining with an intercalating type stain such as ethidium bromide (EtBr), and then irradiating with UV (ultraviolet light).

Such visualization method requires sequentially following 4 steps: preparation of a nucleic acid specimen, gel electrophoresis, staining, and visualization, requiring a long time before acquiring experimental results. In addition, there are costs incurred for having made available a UV irradiation device for visualization along with the equipment for protecting the skin or eyes of an experimenter. Furthermore, intercalate staining agents are alleged to be highly carcinogenic, requiring care in handling.

In addition, the method using EtBr requires a dedicated device equipped with a functional capability of simultaneously carrying out gel staining and UV irradiation so as to permit a real time observation of the mobility of the target nucleic acid specimen to be stained after termination of gel electrophoresis. Therefore, it has been desired, for improved experimental efficiency, to have a method for visualization of nucleic acids which enables visualization in short time and which is simple and highly safe.

In addressing this situation, currently a variety of methods have been proposed in which a labeled substance is linked to a nucleic acid specimen for visualization. For example, non-patented reference 1 (Steve Adkins and Margit Burmeister, "Visualization of DNA in Agarose Gels as Migrating Colored Bands: Applications for Preparative Gels and Educational Demonstrations", Analytical Biochemistry 240, 17-23 (1996)) calls for preliminarily binding a color staining agent such as Nile blue, Methyl green, or the like to a nucleic acid specimen, thereby visualizing the nucleic acid specimen.

However, there still remain problems yet to be solved even with the above method for visualizing a nucleic acid. For example, the method of the non-patent reference 1, in which a nucleic acid specimen and a colored staining agent are bound through a weak electrostatic interaction, limits its application only to gel electrophoresis, which makes this method unsuitable for use in other nucleic acid detection methods. In addition, mixing nucleic acid specimens which are bound with a plurality of staining agents respectively will result in mixing up these staining agents and make it impossible to uniquely stain each nucleic acid specimen in different colors.

In regard to these problems, the present inventors came to a conclusion that it would be useful, for visualizing nucleic acids in a variety of situations efficiently and at low cost, to employ nucleic acid derivatives in which designated compounds such as labeling substrates have been introduced by a specific addition reaction to the nucleic acid specimen.

It is an object of the present invention that addresses such problems as mentioned above, to provide nucleic acid derivatives in which a designated compound is introduced specifically to the thymine base or uracil base, components of nucleic acids; a process for the preparation thereof; and a method for use thereof.

Nucleic acid derivatives have been broadly known as anticancer and antiviral agents, antimetabolites and their efficacy had prompted extensive research and development of novel nucleic acid derivatives. For example, Japanese Patent No. 3,142,874 discloses nucleoside, nucleic acid derivatives usable as antitumor agents in which a 3-position of the sugar moiety of the nucleic acid is substituted.

A method has been previously disclosed for introducing a designated molecule into a base or substituting it with a functional group by Smith et al ("Adenine and Thymine Grafts on Polyethyleneimine", Journal of Polymer Science: Part A: Polymer Chemistry, 27 (2) 575-582 (1989)) thereby introducing a vinyl sulfone group at the N-1 position of a thymine using triethylamine in dimethyl formamide. However, this method calls for using a thymine base with its N-1 position being hydrogen in an anhydrous solvent (organic solvent), making it impossible to modify a thymine base as a constituting component of DNA and RNA (nucleic acids) in which the N-1 position is linked to sugar.

The present inventors searched functional groups which link specifically to a base, a component of a nucleic acid, in aqueous solvent and made a novel finding that a vinyl sulfone group and a sulfatoethyl sulfone group, which is a vinyl sulfone group's precursor, specifically link to the N-3 position of the thymine base and uracil base of a nucleic acid, which after extensive studies and many experiments led to the present invention.

### SUMMARY OF THE INVENTION

In accordance with the first main aspect of this invention, there is provided a nucleic acid derivative wherein a designated compound (R) is introduced at the N-3 position of a thymine base or uracil base represented by the following structural formula represented by the following structural formula (I) 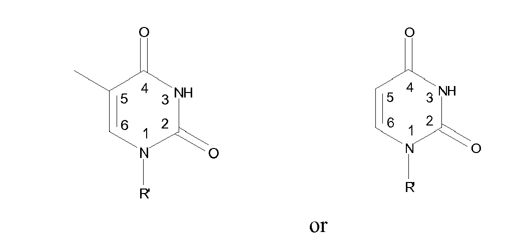 in which (R') at Position 1 is other than hydrogen atom, via a vinyl sulfone group represented by the following structural formula (II); 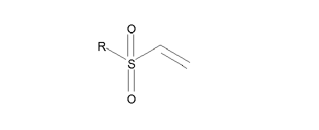 or a sulfatoethyl sulfone group represented by the following structural formula (III).

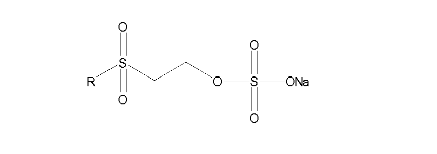

The vinyl sulfone group has been originally used in a reactive dyeing where fibers and the dye chemically react to be linked, particularly, as dyes for cellulosic fibers, but it has never been known that it specifically links to the N-3 position of a nucleic acid base.

Further, the resultant nucleic acid derivatives of the present invention may be used as functional nucleic acid derivatives having a variety of functions by varying the designated compound (R) in accordance with the objective.

In accordance with one embodiment of the present invention there is provided a nucleic acid derivative in which the foregoing designated compound (R) comprises a compound capable of specifically linking to another compound. In accordance with another embodiment, there are provided nucleic acid derivatives wherein the foregoing designated compound (R) is selected from the group consisting of dyes, fluorescent dyes and radioactive isotope labeled substrates. Preferably, the foregoing dyes contain as a chromophore a compound selected from the group consisting of nitro, azo, stilbene, carbonium, quinoline, methine, and anthraquinone. In accordance with still another embodiment the foregoing nucleic acid derivative is used as a nucleic acid molecular weight marker. Such a constitution permits visualization of a nucleic acid.

Further, in accordance with the second main aspect of the present invention, there is provided a nucleic acid molecular weight marker said marker being a DNA or RNA ,wherein a designated compound (R) is linked to the N-3 position of a thymine base or uracil base represented by the following structural formula (IV) 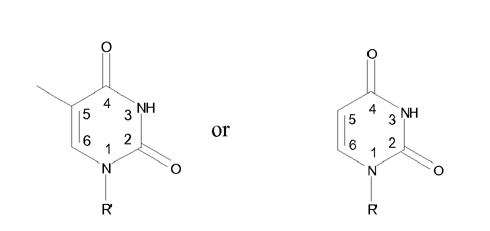 in which (R') at Position 1 is other than hydrogen atom, via a vinyl sulfone group represented by the following structural formula (v); 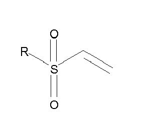 or a sulfatoethyl sulfone group represented by the following structural formula (VI).

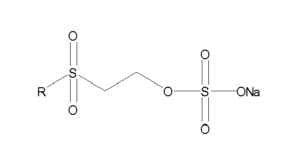

In accordance with one embodiment of the present invention there is provided a nucleic acid molecular weight marker, wherein the foregoing designated compound (R) comprises a compound which can specifically link to another compound. In accordance with still another embodiment, there is provided a nucleic acid molecular weight marker, wherein the foregoing designated compound (R) is selected from the group consisting of dyes, fluorescent dyes and radioactive isotope labeled substrates. Preferably, the foregoing dyes contain as a chromophore a compound selected from the group consisting of nitro, azo, stilbene, carbonium, quinoline, methine, and anthraquinone. Further preferably, the foregoing fluorescent dye is one capable of emitting light in a process in which the chromophore excited by exciting light emits energy. According to such constitution, it is no longer necessary to have a staining step as does the conventional EtBr staining, thereby allowing providing a nucleic acid molecular weight marker which is outstanding in terms of safety and simplicity.

In accordance with the third main aspect of this invention, there is provided a process for the preparation of a nucleic acid derivative into which a designated compound (R) is introduced, said process comprising a step of having a given nucleic acid made available; and a step of linking the designated compound (R) to the N-3 position of a thymine base or uracil base of said nucleic acid via a vinyl sulfone group represented by the following structural formula (VII); 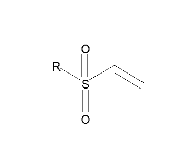 or a sulfatoethyl sulfone group represented by the following structural formula (VIII).

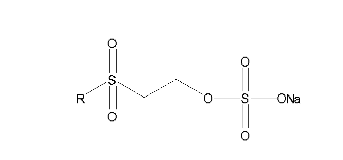

For example, the linking of a designated compound (R) to the N-3 position of the thymine and uracil bases by the method of the present invention results in products with the following structural formulae:

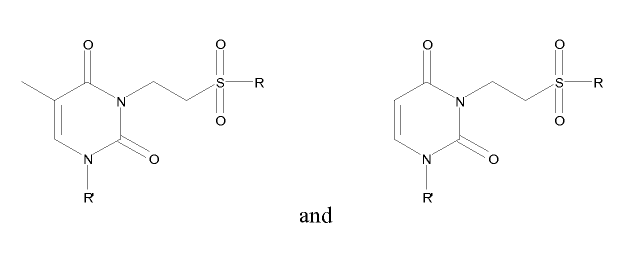

Further, in accordance with one embodiment of the present invention, there is provided a process, wherein the foregoing designated compound (R) comprises a compound which can be linked specifically to another compound. Further, in accordance with another embodiment of the present invention, there is provided a process, wherein the foregoing designated compound (R) is selected from the group consisting of dyes, fluorescent dyes, and radioactive isotope labeled substrates. Preferably, the foregoing dyes contain as a chromophore a compound selected from the group consisting of nitro, azo, stilbene, carbonium, quinoline, methine, and anthraquinone. Further, preferably the foregoing fluorescent dye is capable of emitting light in a process in which the chromophore excited by exciting light emits energy. In accordance with still another embodiment, the foregoing nucleic acid derivative is one used as a nucleic acid molecular weight marker. Such structures allow preparing a nucleic acid derivative that can be used as a visualized nucleic acid molecular weight marker.

Further, in accordance with the fourth main aspect of the present invention, there is provided a method for determining the molecular weight of a target nucleic acid, said method comprising: a step of having made available a nucleic acid derivative of the present invention as a nucleic acid molecular weight marker; a step of having made available the foregoing target nucleic acid and linking a designated compound (R) to the N-3 position of the thymine base or uracil base of the target nucleic acid via a vinyl sulfone group represented by the following structural formula (IX); 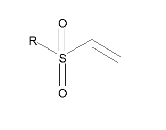 or a sulfatoethyl sulfone group represented by the following structural formula (X); 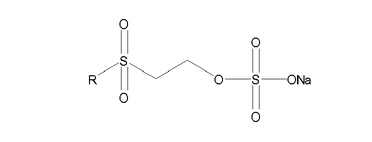 a step of subjecting said target nucleic acid and the nucleic acid molecular weight marker to gel electrophoresis; and a step of comparing the position of said target nucleic acid with the position of said nucleic molecular weight marker, thereby determining the molecular weight of said target nucleic acid.

Further, in accordance with the fifth main aspect of the present invention, there is provided a method for determining the molecular weight of a target nucleic acid, said method comprising: a step of having made available a nucleic acid molecular weight marker of the present invention; a step of having made available the foregoing target nucleic acid and linking a designated compound (R) to the N-3 position of the thymine base or uracil base of the target nucleic acid via a vinyl sulfone group represented by the following structural formula (XI); 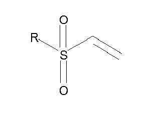 or a sulfatoethyl sulfone group represented by the following structural formula (XII); 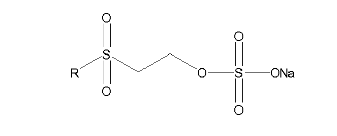 a step of subjecting said target nucleic acid and the nucleic acid molecular weight marker to gel electrophoresis; and a step of comparing the position of said target nucleic acid with the position of said nucleic acid molecular weight marker, thereby determining the molecular weight of said target nucleic acid. Such constitution permits visualizing in real time the mobility of the target nucleic acid.

Further features and significant advantageous effects of the present invention will be made clear to those skilled in the art from the sections on the detailed description of the preferred embodiments described below.

### BRIEF DESCRIPTION OF THE DRAWINGS

Fig. 1 shows electrophoretic images of Poly-dA, dT, dG and dC stained with 5-acetamido - 4-hydroxy-3-[2-hydroxy-4-(sulfoxyethyl)-sulfonyl]-phenylazo-2,7-naphthalenedisulfonic acid copper complex.
Fig. 2 shows an EcoRV digested product (2961 base pairs) of pBluescript II SK(+) stained with 4-amino-N-(3-[vinylsulfonyl] phenyl) naphthalimide-3,6-disulfonate dilithium salt.
Fig. 3 shows an electrophoretic comparison of a stained ladder with an RNA marker.
Fig. 4 shows an electrophoretic comparison of a stained naturally derived DNA ladder with a single strand RNA marker.

### DETAILED DESCRIPTION OF THE PREFERRED EMBODIMENTS

As described above, in accordance with the present invention there are provided nucleic acid derivatives in which a designated compound is introduced specifically into a thymine base or uracil base, components of nucleic acids; a process for the preparation thereof; and a method for use thereof.

The nucleic acid derivatives of the present invention thus obtained may be used as functional nucleic acid derivatives having a variety of functions by varying the designated compound according to the objective.

The designated compound (R) in the present invention may be selected from, but is not limited to, dyes, fluorescent dyes, radioactive isotope labeled substrates, and compounds which can link specifically to other compounds. For example, the fluorescent dyes may be Cy (trademark) 3, Cy (trademark) 5, FITC and the like; the compounds capable of specifically linking to other compounds may be biotin labeling and DIG labeling reagents, and the like. Preferably, the foregoing designated compounds are dyes and/or fluorescent dyes thereby allowing preparation of visualized nucleic acids, one of the objects of the present invention.

More specifically, if a dye such as a color dye is selected as a desired compound of the present invention, this will allow use thereof as a visualized nucleic acid molecular weight marker. Such a constitution will no longer require the conventional staining step such as the EtBr staining and can provide a visualized nucleic acid molecular weight marker, which is much outstanding in terms of safety and simplicity. Since the nucleic acid molecular weight marker of the present invention permits observing the mobility of the target nucleic acid in real time, there is no need to preset experimental conditions, and the electrophoretic time can be adjusted dependent upon the target nucleic acid, so that the duration of the experiment can be shortened. Furthermore, since the foregoing dye is linked specifically and strongly to a thymine base and/or uracil base, this permits preparing nucleic acid molecular weight markers with the type of dye varied for each of multiple nucleic acid fragments, thereby permitting a simple and distinct molecular weight estimate.

For detecting proteins, DNA, and RNA, important bio-polymers, there are important and frequently performed experimental procedures in molecular biological studies with names coined in reference to their directions, such as the Western blot, Southern blot, and Northern blot methods, which all call for electrophoresis followed by transferring electrically or through permeation onto nitro-cellulose or nylon films and detecting the target molecule with high sensitivity using a specific probe. However, among these, there has been a molecular weight marker that permits checking in real time the status of electrophoresis or film transfer under visual light conditions, available only for the Western blot for proteins; but there have been no molecular weight markers for DNA and RNA which permit a direct observation, under visual light, of the status of the electrophoresis or of transfer to films.

It is known that in film transfer experiments, molecular weight greatly affects the transfer efficiency; and up to now the transfer efficiency has been indirectly checked after the transfer by dyeing the DNA or RNA remaining on the gel side. In contrast, the present invention enables one to easily check under visible light without interruption gel electrophoresis and the progress status of the electrophoresis, and also permits the colored DNA, during its transfer to films by the Northern blot or Southern blot or the like, to be transferred to the films and fixated strongly thereto, in the same manner as with an unmodified DNA or RNA. This permits readily observing and checking the transfer status to the films under visible light. Furthermore, it has been found that the transferred and colored molecular weight marker for a DNA or RNA in accordance with the present invention neither hybridizes with a probe, nor generates a background, or interferes with detection by an original hybridization.

In accordance with the present invention, molecular weight markers are now made available which can be visualized under visible light for all the blotting methods: the Western blot, Southern blot, and Northern blot methods.

In addition, if a fluorescent dye is selected as the foregoing designated compound, it is possible to prepare a nucleic acid probe usable for checking the presence or location of a target nucleic acid in cells (in situ).

The nucleic acid derivatives of the present invention can also be used not only as visualized nucleic acids, but can also be used in a hybridization process for linking, to a fixed layer, a nucleic acid derivative of the present invention via the forgoing vinyl sulfone group and/or sulfatoethyl sulfone group, thereby permitting detection of the specific base sequence of a target nucleic acid.

The nucleic acids used in the present invention are not particularly limited, but they include DNA, dsDNA, ssDNA, cDNA, RNA, dsRNA, ssRNA, mRNA, hnRNA, tRNA, rRNA, miRNA, siRAN and the like, suitably selected by those skilled in the art, dependent on their application.

The length of the nucleic acid used in the present invention may be suitably varied, dependent on the application by those skilled in the art. For example, the length of the nucleic acids used in nucleic acid molecular weight markers in accordance with the present invention is 50-1,000bp for use in a low molecular weight determination and 300-10,000bp for a high molecular weight determination.

The process for the preparation of nucleic acid derivatives of the present invention is performed in aqueous media wherein the N-1 position of the thymine base is other than hydrogen atom. This permits determining the molecular weight of a target nucleic acid under experimental conditions close to natural (in vivo) conditions. Use of an aqueous medium provides the advantages of being friendly to the environment and requiring no special equipment such as a local exhaust device and the like. Furthermore, since polymer DNAs and polymer RNAs are insoluble in organic solvents, there is an advantage for facilitating the introduction of a compound thereinto by reacting in an aqueous medium. The aqueous media include, but are not limited to, water, potassium carbonate-potassium chloride solution, and the like.

The nucleic acid specimen in the present invention may be made available using methods known to those skilled in the art, for example, PCR, chemical synthesis, extraction from living organisms, and the like.

The gel electrophoresis used in the present invention may suitably be selected by those skilled in the art, dependent upon the target nucleic acid, such as, for example, modified acrylamide gel electrophoresis, agarose gel electrophoresis, modified agarose gel electrophoresis, and the like. Various gel electrophoresis methods are not particularly limited as long as they are procedures and methods known to those skilled in the art.

The advantageous effects of the present invention are now explained with reference to examples. However, it should be understood that the present invention is not limited to the examples described below, and may be easily altered or modified by those skilled in the art.

### (Examples)

Examples 1 and 2 to be described later were run using the following materials and methods.

### (1) Quantification of DNA

A measurement of UV absorption at 260nm was made of a DNA solution dissolved in appropriate H₂O or 1 x TE. The spectrometer was a U-2800 made by Hitachi Co..

### (2) Modified Acrylamide Gel Electrophoresis

The modified acrylamide gels used were : 10%, 12.5% and 15% polyacryl amide gels containing 7.5M urea; the electrophoresis buffer used was 1 x TBE. The sample buffer used was Loading Buffer PA made by BioDynamics Inc., which was modified by heating at 80°C for 5 minutes and was subjected to electrophoresis. The electrophoresis time was that required for BPB to migrate to a suitable position at 200V

### (3) Agarose Gel Electrophoresis

Agarose gel electrophoresis was carried out with a 0.8% agarose gel. The electrophoresis buffer was 1 x TAE and 1 x TBE. The sample buffer was 6 x BPB made by BioDynamics Inc., which was modified with heating at 75°C for 3 minutes and then subjected to electrophoresis. The electrophoresis time was that required for BPB to migrate to a suitable position at 5 cm/5V

### (4) Modified agarose gel

Modified electrophoresis was carried out with 1.0% or 1.5% agarose gels containing 2.2M formaldehyde. The electrophoresis buffer used was 1 x TBE. The sample buffer used was a Loading Buffer AG+ made by BioDynamics Inc. The electrophoresis time was that required for BPB to migrate to a suitable position at 5 cm/V

### (5) Determination after Electrophoresis

The polyamide and agarose gels after electrophoresis were stained with aqueous ethidium bromide (EtBr) solution or aqueous crystal violets (CV) solution. The EtBr staining was carried out by immersing the gel for 15 minutes in an aqueous 7µg/ml EtBr solution and observing a fluorescent coloration by UV irradiation on a transilluminator. The CV staining was carried out by immersing the gel in aqueous 20µg/ml CV solution and observing by visual inspection under the visual light.

### (6) Removal of Dyes

The unreacted dyes after DNA was subjected to a staining reaction were removed on a G25 spin column made by GE-Healthcare Bioscience Corp. The unused spin column was centrifuged at 2700 x g for 1 minute thereby removing excess buffer, followed by applying approximately 50-100µl of a staining reaction solution to the column and by centrifuge elution at 2700 x g for 2 minutes.

### (7) Thin Layer Chromatography

Thin layer chromatography (TLC) was carried out using a Silica-Gel-60F254 made by Merck Company. TLC spots were detected at a wavelength 254 nm. The coloration agent used was an ammonium molybdate-cerium alum solution.

### (8) Nuclear magnetic resonance

Nuclear magnetic resonance (NMR) spectra were measured for ¹H-NMR at 500MHz using an ECA-500 FT-NMR made by JEOL Ltd. The solvent used for the measurement was deuterated chloroform. The standard reference used was tetramethylsilane TMS.

### (9) Mass Spectrum

Mass spectroscopy (MS) was carried out using an M-2500 Gas Chromatograph-mass spectrometer made by Hitachi Co. Measurement was made with a direct injection EI method.

### [Example 1]

Experiments were carried out in this example to introduce a designated compound using a vinyl sulfone group and a sulfatoethyl sulfone group.

### (1) Introduction of a dye having a sulfatoethyl sulfone group into 30 mer poly-dA, dG, dT, dC and dU.

### (Method)

To each 12.5µl of a DNA solution, obtained by dissolving chemically synthesized DNA Oligomers (poly-dA, dG, dT, dC and dU) (Table 1) in H₂O to a concentration of 0,4µg/µl, was added 12.5µl of a dye solution of the following 5-acetamido -4-hydroxy-3-[2-hydroxy-4-(sulfoxyethyl)-sulfonyl]-phenylazo-2,7-naphthalenedisulfonic acid copper complex having a sulfatoethyl sulfone group, dissolved in 0.2M K₂CO₃-0.8M KCl, pH12 to a concentration of 20µg/µl, followed by reacting on a heat block at 70°C for 1.5 hours.

**Table 1 Sequence of 30mer poly-dA, dG, dT, dC and dU**

| Name | Sequence |
|---|---|
| Poly-dA | AAAAAAAAAAAAAAAAAAAAAAAAAAAAAA |
| Poly-dG | GGGGGGGGGGGGGGGGGGGGGGGGGGGGGG |
| Poly-dT | TTTTTTTTTTTTTTTTTTTTTTTTTTTTTT |
| Poly-dC | CCCCCCCCCCCCCCCCCCCCCCCCCCCCCC |
| Poly-dU | UUUUUUUUUUUUUUUUUUUUUUUUUUUUUU |

| | |
|---|---|
| * All are deoxyribonucleic acids. | |

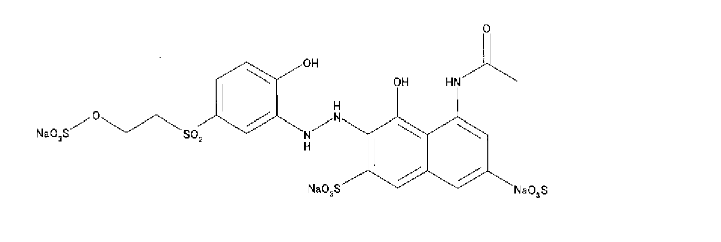

The reacted solution was purified on a G25 spin column (GE-Healthcare Bioscience Corp, and the eluted solution was concentrated to dryness by freeze drying. The resultant pellets were redissolved by adding 1 x TE (pH8) buffer and examined by electrophoresis using a modified acrylamide gel.

### (Result)

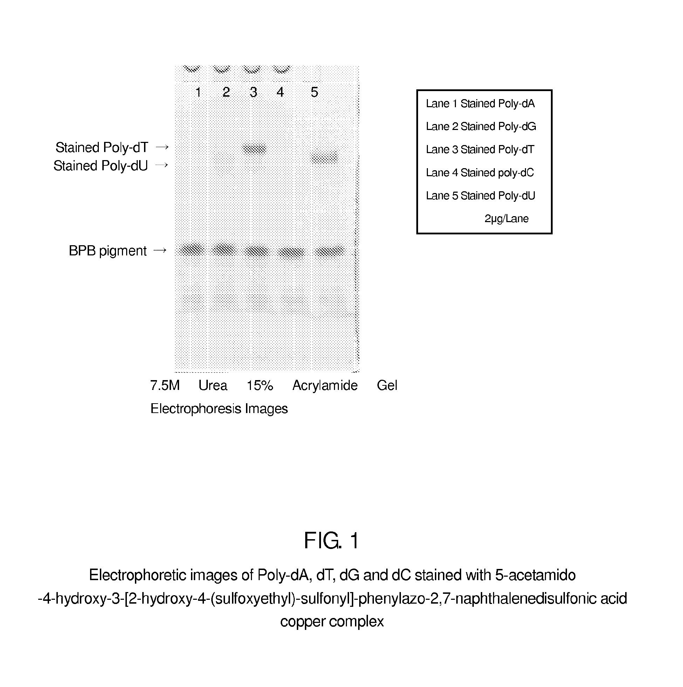

The above experiment allowed determining that 5-acetamido -4-hydroxy-3-[2-hydroxy-4-(sulfoxyethyl)-sulfonyl]-phenylazo-2,7-naphthalenedisulfonic acid copper complex, a reactive dye molecule having a sulfatoethyl sulfone group, stained only Poly-dT and Poly-dU, but not Poly-dA, Poly-dG or Poly-dC (Fig. 1).

This shows that the sulfatoethyl sulfone group undergoes an addition reaction specifically with the thymine or uracil base moieties, but not with the adenine base, guanine base, cystine base or further with the deoxyribose or phosphate moieties. This demonstrates that the specific addition reaction of a sulfatoethyl sulfone group with a thymine base and with a uracil base can introduce a designated compound into any molecules containing these bases, for example, natural DNA, RNA and the like.

Introduction of a fluorescent dye having a vinyl sulfone group into a naturally derived double strand DNA.

### (Method)

To 5 µl of a DNA solution obtained by dissolving in H₂O to a concentration 1µg/µl of a naturally derived linear double strand DNA having 2,961 base pairs, an EcoRV digested product of Plasmid DNA, pBluescript II SK(+), was added a 5µl dye solution of a reactive fluorescent reagent having a vinyl sulfone group, the following 4-amino-N-(3-[vinylsulfonyl] phenyl) naphthalimide-3,6-disulfonate dilithium salt, dissolved in 0.2M K₂CKO₃, 0.8M KCl (pH12) to a concentration of 40µg/µl, followed by reacting for 3 hours at 65°C on a heat block.

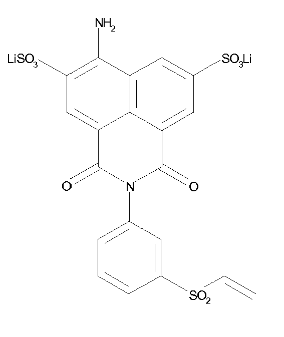

Three hours later the reaction was terminated by adding 5µl of 1N Tris-HCL (pH9); the reacted solution was then purified on a G25 spin column (GE Healthcare Bioscience Corp). H₂O was added to bring the eluted solution to 0.1µg DNA/µl, followed by subjecting a 5µl or 1µl portion to electrophoresis on an agarose gel.

### (Result)

The above experiment shows that a natural DNA can also be fluorescence stained by a specific addition reaction, via the vinyl sulfone group in accordance with the present invention, to the thymine base in DNA, with a reactive fluorescence reagent having a vinyl sulfone group:

4-amino-N-(3-[vinylsulfonyl] phenyl) naphthalimide-3,6-disulfonate dilithium salt. The specific addition reaction of a vinyl sulfone group with a thymine base by the present method shows it to be useful as a new method for introducing a designated compound such as a fluorescent reagent and the like into a natural DNA.

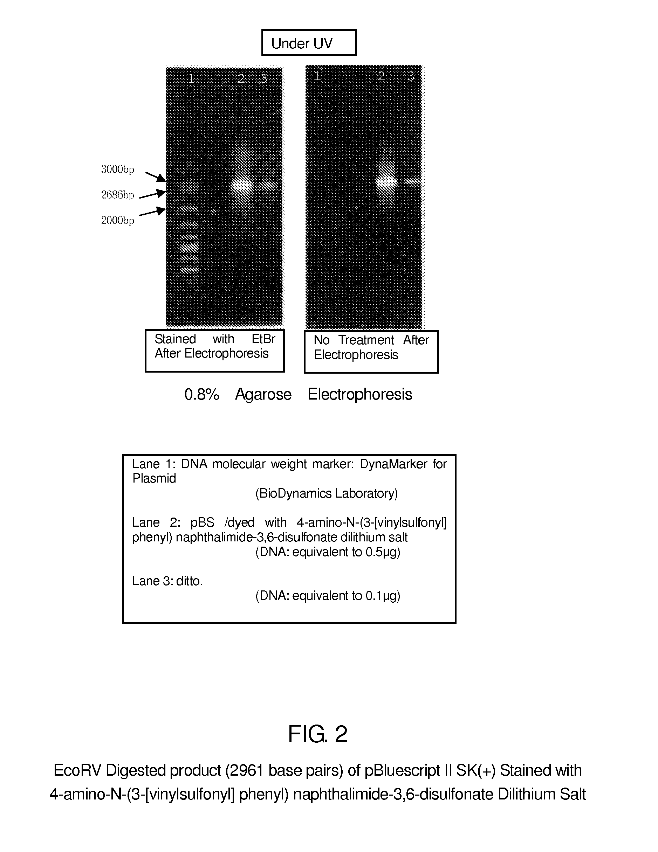

In addition, the fluorescence modified DNA in accordance with the present invention undergoes an electrophoretic migration on agarose gel electrophoresis to a position of about 3000 bases, which is nearly identical to that of an intact DNA as unmodified (Fig. 2).

### (3) Introduction of a substance having a vinyl sulfone group into a thymine base.

### (Method)

1-methylthymine represented by the following structural formula was used as a model for thymine and uracil bases, constituting components of DNA and RNA;

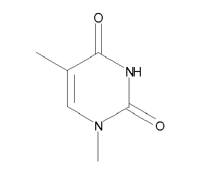

In addition, p-Tolyl vinyl sulfone represented by the following structural formula was used as a compound having a vinyl sulfone group.

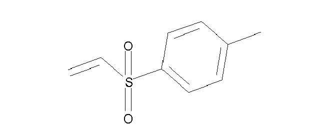

20.5 mg of 1-methylthymine (MW=140.14) was suspended in 0.1M K₂CO₃-0.4M KC1 (pH12), followed by adding 40.0mg of p-tolyl vinyl sulfone (MW=182.24) and reacting for 1 hour at 70°C on a heat block. At the end of reaction, chloroform was added and the mixture was stirred, and the organic layer was separated. The aqueous layer was extracted once with chloroform and the resultant organic layer was washed with aqueous 0.1N HCl solution, water, and aqueous saturated sodium chloride, dried over anhydrous sodium sulfate, and concentrated at reduced pressure. The resultant crystals were purified by thin layer silica gel chromatography (Et₂O: AcONa = 1:1) yielding 35.2mg of the goal product having the following structural formula: 1-methyl-3-(p-tolyl sulfonylethyl) thymine; (Rf= 0.36, Et₂O: AcONa= 1:1) at a 75% yield.

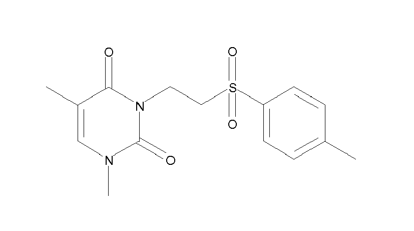

The resultant crystals were subjected to ¹H-NMR and MS spectroscopy for determining the structure.

### (Result)

The above experiment showed that the 1-methyl-3-(p-tolyl sulfonylethyl) thymine had [M]⁺ observed by MS spectrometry at m/z = 322; three methyl group signals observed near 1.84ppm, 2.42ppm, and 3.30ppm in an ¹H-NMR as shown in Table 2; and two correlative methylene signals observed near 3.45ppm and 4.25ppm. It was determined that with the disappearance of a broad N-3 proton signal derived from 1-methylthymine near 8.25ppm, the product was such that the 1-methylthymine was coupled with p-tolyl vinyl sulfone with the mode of coupling as that of linking the N-3 position of 1-methylthymine to the p-tolyl group via the ethyl sulfone.

### [Table 2]

**Table 2 ¹H-NMR (500 MHz, CDC13)**

| ppm | Signals | Number of Hydrogen atoms |
|---|---|---|
| 1.84 | singlet | 3H |
| 2.42 | singlet | 3H |
| 3.30 | singlet | 3H |
| 3.45 | triplet (4Hz) | 2H |
| 4.25 | triplet (4Hz) | 2H |
| 6.94 | singlet | 1H |
| 7.34 | doublet (8Hz) | 2H |
| 7.80 | doublet (8Hz) | 2H |

This reaction is a typical model for the linking of a thymine base to a vinyl sulfone type reactive dye having a sulfatoethyl sulfone group or to a fluorescent dye having a vinyl sulfone group, showing that the visualization or fluorescence staining of a DNA, poly-dT and Poly-dU results from the addition of the vinyl sulfone group of the reactive dye to the N-3 position of the thymine base and to the uracil base which is similar in the reaction site.

The present method shows it is a useful one for introducing any designated compound via ethyl sulfone under mild conditions, at high yield and with high specificity to any substance having a thymine or a similarly structured uracil base.

### [Example 2]

This example verified the usefulness of a dyed nucleic acid strand as a marker for molecular weight on gel electrophoresis.

### (1) Preparation of a visualized DNA ladder using chemically synthesized Poly-dTs.

### (Method)

To 5µl of a solution dissolved in H₂O to a concentration of 1µg/µl from each of chemically synthesized Poly-dT strands (50, 40, 30, and 20 basis) was added a 5µl solution of the following 2-anthracene-sulfonic acid, 1-amino-9,10-dihydro-9,10-dioxo-4-((3-)(2-) sulfoxy) ethyl) sulfony) phenyl) amino)-disodium salt, dissolved in 0.2M K₂CO₃ (pH12) to a concentration of 20µg/µl, followed by heating at 70°C on a heat block.

### [Table 3]

**Table 3 Poly-dT Strand Sequences**

| Name | Sequence |
|---|---|
| 50 Base-Poly-dT | TTTTTTTTTTTTTTTTTTTTTTTTTTTTTTTTTTTTTTTTTTTTTTTTTT |
| 40 Base-Poly-dT | TTTTTTTTTTTTTTTTTTTTTTTTTTTTTTTTTTTTTTTT |
| 30 Base-Poly-dT | TTTTTTTTTTTTTTTTTTTTTTTTTTTTTT |
| 20 Base-Poly-dT | TTTTTTTTTTTTTTTTTTTT |

| | |
|---|---|
| * All are deoxyribophosphates. | |

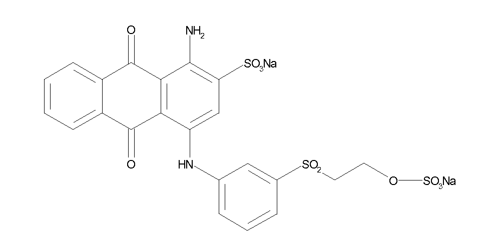

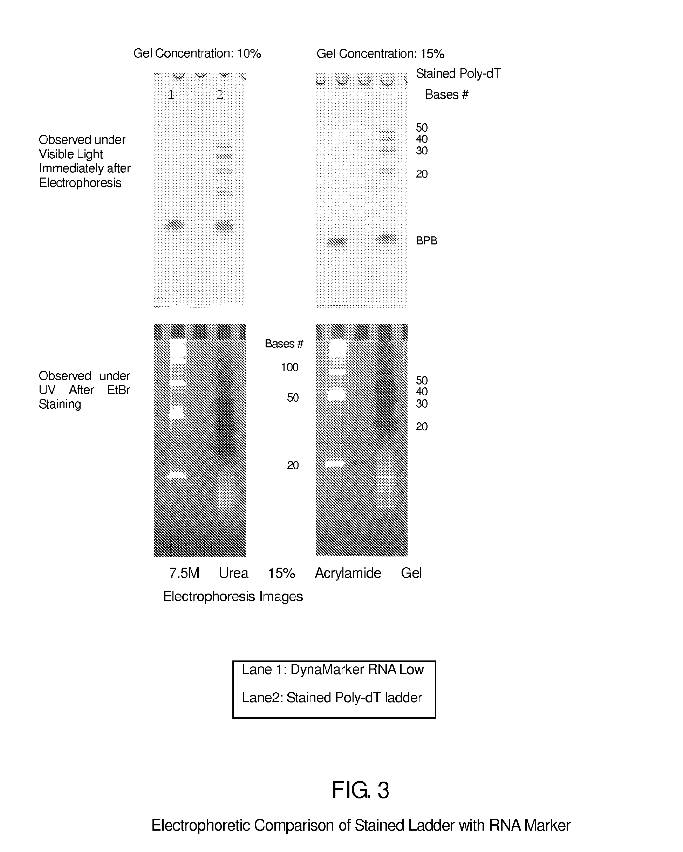

Thirty minutes later, 5µl of a dye solution, dissolved to a concentration of 20µg/µl in 0.1M K₂CO₃ (pH12), was added followed by heating for 30 minutes. This was repeated twice to allow the reaction to take place for a total of 1.5 hours. After the reaction, the reacted solution was then purified on a G25 spin column. The eluted solution was concentrated to dryness by freeze drying; 2.8µg of the resultant pellets were redissolved by addition of a buffer, 1 x TE (pH8), to bring it to 2.8µg of DNA/µl followed by mixing at the compositions (Table 4) given below and checking by modified acrylamide gel electrophoresis (Fig. 3).

### [Table 4]

**Table 4 Dyed Ladder (50, 40, 30, 20 base-) 1 Load Formulation**

| Dyed DNA Fragments | Volume |
|---|---|
| Stained 50 Base | 0.25 µl |
| Stained 40 Base | 0.25 µl |
| Stained 30 Base | 0.25 µl |
| Stained 20 Base | 0.25 µl |
| Loading Buffer PA | 4 µl |

### (Result)

The above experiment showed that the apparent molecular weights of the stained Poly-dT ladder (50, 40, 30, and 20 base-) were independent of polyacrylamide gel concentration, with essentially no change in relative positional relationship with each band of a single strand RNA marker (DynaMarker RNA Low made by BioDynamics Inc.). This demonstrates that introduction of a dye via a sulfatoethyl sulfone group in accordance with the present method turns a single strand nucleic acid containing a thymine base into a molecular weight marker for electrophoresis enabling a single strand RNA to be visible under visible light.

### (2) Preparation of a visualized DNA ladder using plasmid DNA.

### (Method)

To a 5µl solution of a DNA solution obtained by dissolving in H₂O restriction enzyme digests (3000, 1500, and 700 base pairs) derived from a plasmid to a concentration of 1µg/µl was added a 5µl dye solution of the following 2,7-naphthalene disulfonic acid 3,6-(bis (4- ((2-hydroxyethyl) sulfonyl) phenyl) bis (azo)-5-amino-4-hydroxy-, di (hydrogen sulfate) ester, tetrasodium salt, dissolved in 0.2M K₂CO₃, 0.8 M KCl (pH12) to a concentration of 40µg/µl, followed by reacting at 70°C on a heat block for three hours.

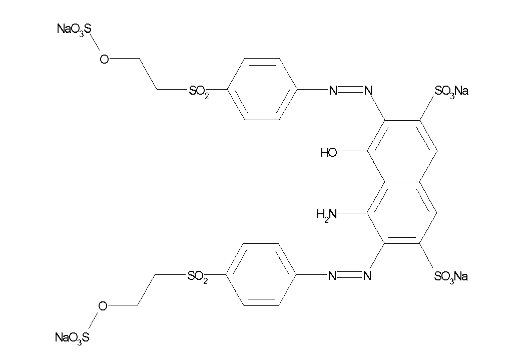

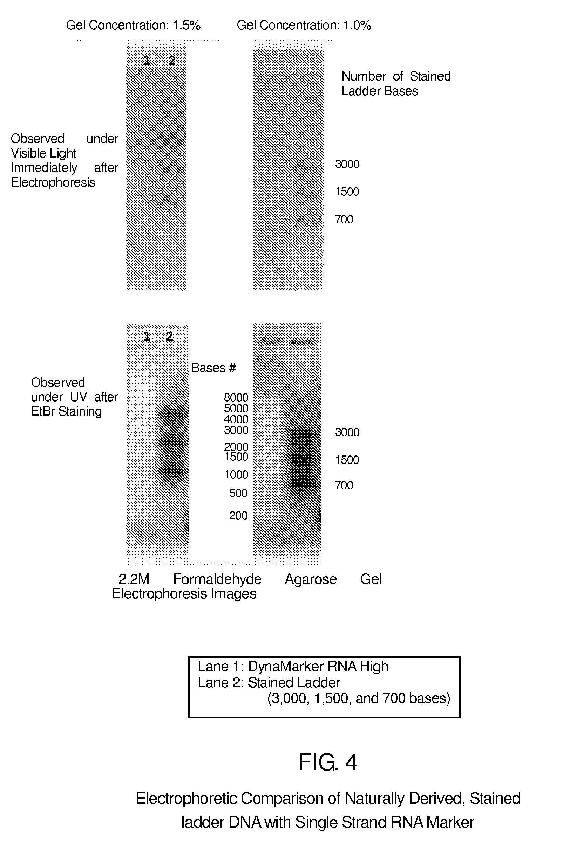

Three hours later the reacted solution was purified on a G25 spin column. The eluted solution was concentrated to dryness by freeze drying; the resultant pellets were redissolved by addition of a 1 x TE buffer to a concentration of a 2.0 µg DNA/µl. The individual stained DNA fragments and Loading Buffer AG+, made by BioDynamics Inc., were mixed as in Table 5 to prepare a stained ladder with 3000, 1500, and 700 bases. This ladder was compared by electrophoresis on agarose gels with an RNA marker made by BioDynamics Inc. (DynaMarker RNA High) (Fig. 4).

### [Table 5]

**Table 5 Stained Ladder (3000, 1500, and 700 base-) 1 Load Portion Formulation**

| Stained DNA Fragments | Volume |
|---|---|
| Stained 3000 Base- | 0.25 µl |
| Stained 1500-700 Base- | 0.5 µl |
| 1xTE | 0.25 µl |
| Loading Buffer AG+ | 4 µl |

### (Result)

The above experiment demonstrates that a visible light dye can also be introduced into a naturally derived DNA by a specific addition reaction of a reactive dye having a sulfatoethyl sulfone group, the following 2,7-naphthalene disulfonic acid 3,6-(bis (4- ((2-hydroxyethyl) sulfonyl) phenyl) bis (azo)-5-amino-4-hydroxy-, di (hydrogen sulfate) ester, tetrasodium salt, via the sulfatoethyl sulfone group in accordance with the present method, to the thymine base in a natural DNA (Fig. 4).

It was demonstrated that the specific addition reaction, in accordance with the present method, of a sulfatoethyl sulfone group to a thymine base is useful as a new procedure for introducing any designated compound such as visible light dyes and the like into a natural DNA.

In addition, the above experiments demonstrated that the apparent molecular weights of stained DNA ladder 3000, 1000, and 700 bases were independent of the polyacrylamide gel concentration; on comparison by electrophoresis of a single strand RNA markers (made by BioDynamics Inc. DynaMarker RNA High), their apparent molecular weights were always at the same positions as those corresponding to the single strand RNAs of the 3000, 1000, 700 bases. Furthermore, in view of experimental results of the stained DNA ladder of the foregoing chemically synthesized Poly-dTs, the stained DNA ladder obtained by the present method functions as a marker for molecular weight in electrophoresis.

## Claims

1. A nucleic acid derivative wherein a designated compound (R) is introduced at the N-3 position of a thymine base or uracil base represented by the following structural formula I, 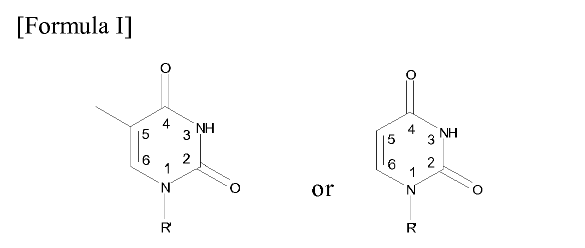 in which (R') at Position 1 is other than hydrogen atom, via a vinyl sulfone group represented by the following structural formula (II) 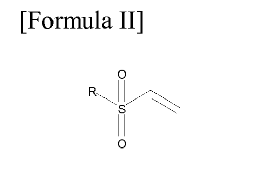 or a sulfatoethyl sulfone group represented by the following structural formula (III).

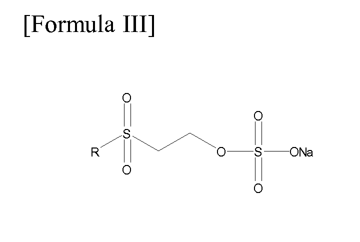

2. The nucleic derivative as set forth in Claim 1, wherein said designated compound (R) comprises a compound which can link specifically to another compound.

3. The nucleic acid derivative as set forth in Claim 1, wherein said designated compound (R) is selected from the group consisting of dyes, fluorescent dyes, and radioactive isotope labeled substrates.

4. The nucleic acid derivative as set forth in Claim 3, wherein said dyes contain as a chromophore a compound selected from the group consisting of nitro, azo, stilbene, carbonium, quinoline, methine, and anthraquinone.

5. The nucleic acid derivative as set forth in Claim 1, wherein said nucleic acid derivative is used as a nucleic acid molecular weight marker.

6. A nucleic acid molecular weight marker, said marker being a DNA or RNA, wherein a designated compound (R) is linked to the N-3 position of a thymine base or uracil base represented by the following structural formula (IV), 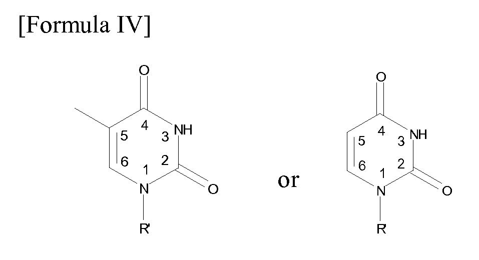 in which (R') at Position 1 is other than hydrogen atom, via a vinyl sulfone group represented by the following structural formula (V) 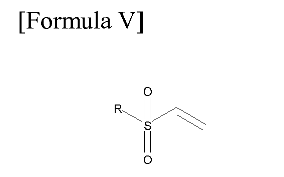 or a sulfatoethyl sulfone group represented by the following structural formula (VI). [Formula VI]

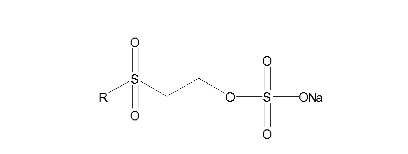

7. The nucleic acid molecular weight marker as set forth in Claim 6, wherein said designated compound (R) comprises a compound which can specifically link to another compound.

8. The nucleic acid molecular weight marker as set forth in Claim 6, wherein said designated compound (R) is selected from the group consisting of dyes, fluorescent dyes, and radioactive isotope labeled substrates.

9. The nucleic acid molecular weight marker set forth in Claim 8, wherein said dyes contain as a chromophore a compound selected from the group consisting of nitro, azo, stilbene, carbonium, quinoline, methine, and anthraquinone.

10. The nucleic acid molecular weight marker as set forth in Claim 8, wherein said fluorescent dye is capable of emitting light in a process in which the chromophore excited by exciting light emits energy.

11. A process for the preparation of a nucleic acid derivative into which a designated compound (R) is introduced, said process comprising
a step of having a given nucleic acid made available; and
a step of linking the designated compound (R) to the N-3 position of a thymine base or uracil base of said nucleic acid via a vinyl sulfone group represented by the following structural formula (VII), 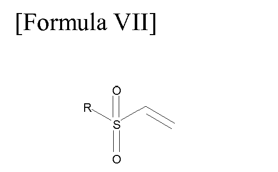
or a sulfatoethyl sulfone group represented by the following structural formula (VIII).

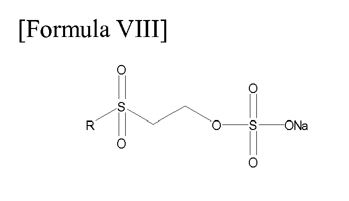

12. The process as set forth in Claim11, wherein said designated compound (R) comprises a compound which can link specifically to another compound.

13. The process as set forth in Claim11, wherein said designated compound (R) is selected from the group consisting of dyes, fluorescent dyes, and radioactive isotope labeled substrates.

14. The process as set forth in Claim13, wherein said dyes contain as a chromophore a compound selected from the group consisting of nitro, azo, stilbene, carbonium, quinoline, methine, and anthraquinone.

15. The process set forth in Claim13, wherein said fluorescent dye is capable of emitting light in a process in which the chromophore excited by exciting light emits energy.

16. The process as set forth in Claim11, wherein said nucleic acid derivative is used as a nucleic acid molecular weight marker.

17. A method for determining the molecular weight of a target nucleic acid, said method comprising:
a step of having made available the nucleic acid derivative as set forth in Claim 1 as a nucleic acid molecular weight marker;
a step of having made available the foregoing target nucleic acid and linking a designated compound (R) to the N-3 position of the thymine base or uracil base of the target nucleic acid via a vinyl sulfone group represented by the following structural formula (IX) ; 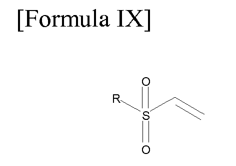 or a sulfatoethyl sulfone group represented by the following structural formula (X); 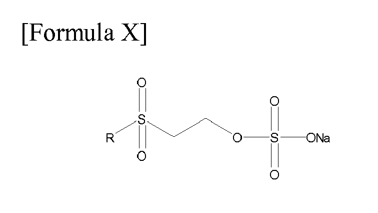
a step of subjecting said target nucleic acid and the nucleic acid molecular weight marker to gel electrophoresis; and
a step of comparing the position of said target nucleic acid with the position of said nucleic acid molecular weight marker, thereby determining the molecular weight of said target nucleic acid.

18. A method for determining the molecular weight of a target nucleic acid, said method comprising:
a step of having made available the nucleic acid molecular weight marker as set forth in Claim 6;
a step of having made available said target nucleic acid and linking a designated compound (R) to the N-3 position of the thymine base or uracil base of the target nucleic acid via a vinyl sulfone group represented by the following structural formula (XI); 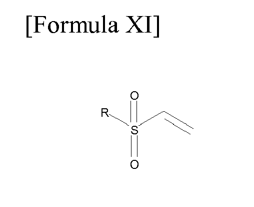 or a sulfatoethyl sulfone group represented by the following structural formula (XII); 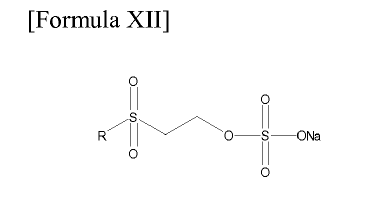
a step of subjecting said target nucleic acid and the nucleic acid molecular weight marker to gel electrophoresis; and
a step of comparing the position of said target nucleic acid with the position of said nucleic acid molecular weight marker, thereby determining the molecular weight of said target nucleic acid.
